# EUROPEAN PATENT SPECIFICATION

(11) **EP 1 641 429 B1**
(45) Date of publication and mention of the grant of the patent: **13.08.2008**
(21) Application number: 04743237.2
(22) Date of filing: 05.07.2004
(51) Int. Cl.: A61K 8/18, C08K 3/22, C08K 3/00, C09D 7/12, D06M 11/44, D06M 11/46, G03C 1/815

(54) **METAL OXIDE FORMULATIONS**
METALLOXID-FORMULIERUNGEN
FORMULATIONS D'OXYDE METALLIQUE

(30) Priority: 03.07.2003 GB 0315656
(43) Date of publication of application: 05.04.2006
(73) Proprietor: Oxonica Limited, Kidlington, Oxford OX5 1PF (GB)
(72) Inventor: PARK, George Barry, Oxonica Limited, Kidlington, Oxford OX5 1PF (GB); FLUTTER, Barry, Oxonica Limited, Kidlington, Oxford OX5 1PF (GB); KNOWLAND, John, Oxonica Limited, Kidlington, Oxford OX5 1PF (GB)
(74) Representative: Benson, John Everett
(86) International application number: PCT/GB2004/002891
(87) International publication number: WO 2005/002538

(56) References cited:
- EP-A- 0 526 712
- WO-A-01/40114
- WO-A-99/60994
- WO-A-03/083008
- WO-A-20/04058209
- WO-A-20/04105487
- US-A- 3 293 037
- US-A- 3 317 321
- US-A- 5 441 726
- US-A- 5 736 308
- US-A1- 2001 039 308
- US-B1- 6 436 374
- PATENT ABSTRACTS OF JAPAN vol. 2000, no. 09, 13 October 2000 (2000-10-13) & JP 2000 169339 A (POLA CHEM IND INC), 20 June 2000 (2000-06-20)
- PATENT ABSTRACTS OF JAPAN vol. 2000, no. 01, 31 January 2000 (2000-01-31) & JP 11 279019 A (SHISHEIDO CO LTD), 12 October 1999 (1999-10-12)

## Description

The present invention relates to metal oxide formulations and especially UV screen compositions including those suitable for cosmetic and topical pharmaceutical use containing such oxides as well as polymeric compositions containing the same.

The effects associated with exposure to sunlight are well known. Thus exposure of the skin to UVA and UVB light may result in, for example, sunburn, premature ageing and skin cancer.

Commercial sunscreens generally contain components which are able to reflect and/or absorb UV light. These components include, for example, inorganic oxides such as zinc oxide and titanium dioxide as well as organic sunscreen agents.

Most organic sunscreen agents absorb light over only a part of the UVA-UVB spectrum with the result that if one is to obtain a screening effect covering the whole UVA-UVB spectrum it is generally necessary to use a combination of different organic sunscreen agents. Some organic sunscreen agents and other components of sunscreen compositions are stable to UV light but others are photosensitive and/or may after being excited by UV light degrade another ingredient of the composition.

Titanium dioxide and zinc oxide are generally formulated as "micronised" or "ultrafine" (20-50 nm) particles (so-called microreflectors) because particles whose size is less than 10% of the wavelength of the incident light scatter light according to Rayleigh's Law, whereby the intensity of scattered light is inversely proportional to the fourth power of the wavelength. Consequently, they scatter UVB light (with a wavelength of from 280 or 290 to315/ 320 nm) and UVA light (with a wavelength of from 315/320 to 400 nm) more than the longer, visible wavelengths, preventing sunburn whilst remaining invisible on the skin.

However, titanium dioxide and zinc oxide also absorb UV light efficiently, leading via the initial formation of electron hole pairs to the formation of superoxide and hydroxyl radicals and which may in turn initiate damage to other components of the composition. The crystalline forms of TiO₂, anatase and rutile, are semiconductors with band gap energies of about 3.23 and 3.06 eV respectively, corresponding to light of about 385 nm and 400 nm (1 eV corresponds to 8066 cm⁻¹). Thus these oxides while providing good wavelength cover can enhance the degradation of organic sunscreen agents, including UVA organic sunscreens, for example oxybenzone as well as cause degradation of other components of the formulation. Attempts have been made to reduce the adverse effects of TiO₂ and ZnO by coating, but coatings are not invariably effective.

WO 2004/058209 describes a UV sunscreening composition comprising one or more organic components which are photosensitive and/or which are degraded and/or in which degradation is induced by another ingredient of the composition, and TiO₂ and/or ZnO which has been doped with another element and/or reduced ZnO.

UV screening compositions comprising doped TiO₂ or ZnO are also described in WO 01/40114.

US 5441726 describes a sunscreen composition comprising rod shaped ZnO particles which may be doped.

It has now surprisingly been found, according to the present invention, that the degradation of any compound which is adversely affected by TiO₂ and/or ZnO, and especially of organic sunscreen agents, can be retarded if the compositions containing them contain zinc oxide or titanium dioxide which has been doped with another element and/or reduced zinc oxide in addition to the "ordinary" TiO₂ and/or ZnO. In other words by using, in a sunscreen composition etc., these doped or reduced materials rather than ordinary titanium dioxide or zinc oxide alone it is, for example, possible either to provide a composition which gives better protection against UV light for the same quantity of organic sunscreen agent or a composition having the same screening effect against UV light but containing a smaller quantity of organic sunscreen agent. Indeed it is possible to provide all day protection sunscreens by incorporating the doped and/or reduced materials.

Accordingly the present invention provides a composition which comprises at least one ingredient which is adversely affected by the presence of TiO₂ and/or ZnO (usually of course the adverse effect will be brought about by UV light in the presence of TiO₂ and/or ZnO), TiO₂ and/or ZnO which has been doped with another element and/or reduced zinc oxide, and contains TiO₂ and/or ZnO which has not been doped or reduced.

Whether or not an adverse effect occurs, and under what conditions, will in general be clear to the person skilled in the art in the given context. Whether or not an effect is adverse, and the conditions that are relevant, might well be different for different products and for different end uses. For example, for sunscreens and other compositions for cosmetic and topical use on the body, one might be concerned with adverse effects to ingredients that arise when the composition is subjected for say 8 hours to UV light of a wavelength from 290 to 400 nm at an intensity corresponding to midday Mediterranean sunlight, or say an intensity of 10 mW per square cm. and in the presence of TiO₂ and/or ZnO. In the case of compositions that are not used on the body, such as paints and coatings, adverse effects on ingredients that become apparent only after exposure of the composition over longer periods of time (for example one week, one month or one year), or under harsher conditions, might well be of concern. In preferred embodiments we are concerned with ingredients that undergo any chemical change (generally a chemical change that renders the composition less functionally effective, or that reduces its useful life) when subjected to the conditions referred to above.

Components which are adversely affected by TiO₂ and/or ZnO are in general those which are sensitive to free radical attack. They may be intrinsically stable; this attack is related to the homolytic bond dissociation energy. Such components include large molecules such as polymers as well as small molecules such as those with ethylenic unsaturation or those which possess a labile hydrogen atom, for example a tertiary hydrogen atom or other labile species including chlorine. Free radical attack may also break amide or ester linkages of small molecules or of large molecules such as polyamides or polyesters.

The presence of TiO₂ or ZnO may result in a change in a physical property of the component. With a polymer this may be, for example, a change in tensile strength or elongation at break; while with a small molecule, the free radical attack generally results in a change in its chemical structure which gives rise to a change in physical properties such as melting point, boiling point, viscosity, a change in its functional character or, in some cases, toxicity. All these changes can, of course, be measured as one skilled in the art would appreciate.

The present invention has particular applicability for UV sunscreen compositions suitable for cosmetic or pharmaceutical use. By "UV sunscreen composition suitable for cosmetic or topical pharmaceutical use" is meant any cosmetic or topical pharmaceutical composition having UV sunscreen activity i.e. it includes compositions whose principal function may not be sunscreening. It will be appreciated that the doped TiO₂/ZnO or reduced ZnO may be the only ingredient of the composition having UV sunscreening activity i.e. the composition need not necessarily contain an organic UV sunscreen agent. However the composition will contain an ingredient which is adversely affected by TiO₂ and/or ZnO. It is to be understood that the composition also contains TiO₂ and/or ZnO which has not been doped or reduced.

The organic component which may be degraded is generally a UV sunscreen agent. Certain components, typically organic sunscreen agents, when exposed to UV light, are substantially stable to this light but are degraded when exposed to UV light in the presence of titanium dioxide or zinc oxide. Accordingly a full spectrum sunscreen agent cannot be satisfactorily formulated using such a combination. If, however, a doped titanium dioxide or a doped or reduced zinc oxide is used (partially or completely) instead of ordinary TiO₂/ZnO the organic sunscreen agent is not degraded in the same way. Thus the present invention also provides a method of increasing the UV spectrum of a sunscreen formulation which comprises an organic sunscreen agent which is adversely affected by UV light in the presence of titanium dioxide and/or zinc oxide which comprises incorporating in the formulation doped TiO₂ and/or doped or reduced ZnO, as well as a composition which comprises an organic sunscreen agent which is adversely affected by UV light (in the presence of undoped TiO₂ and/or ZnO), doped TiO₂ and/or doped or reduced ZnO, and TiO₂ and/or ZnO which han not been doped or reduced.

In a preferred embodiment the composition has a rate of loss of UV absorption due to free radical attack derived from TiO₂ and/or ZnO which is less, preferably at least 5% less, than that of a composition having the same formulation except that it does not contain the said TiO₂ and/or ZnO which has been doped with another element or reduced zinc oxide. Thus if the rate of loss of UV absorption (during UV exposure) over at least a proportion of the UVA and/or UVB spectrum is X then the amount of the organic component(s) which is degraded possesses a said rate of loss of Y where Y is greater than X, preferably by at least 5%, and the amount of doped TiO₂ and/or ZnO and/or reduced zinc oxide reduces the said rate of loss from Y to X.

However, other organic components may also be susceptible to free radical attack, the degraded products that result potentially causing degradation of the UV sunscreen agent.

However, it should be noted that this principle may be applied to compositions other than cosmetics. Where there is present within the composition a specific organic component which is not degraded by UV light, but which is degraded by free radical attack when in contact with TiO₂ and/or ZnO in the presence of UV light, the change in that organic component may be followed by determining a change in one or more physical properties of the composition. Techniques specific to the physical property may be used to follow the change. Such physical properties may include viscosity, melting range and boiling range.

The rate of loss of absorption can be determined by illuminating a sample of the composition with and without the doped TiO₂ and/or ZnO of defined thickness with UV light of the appropriate wavelength, and determining the absorption of UV light by the composition over a given period, typically 60 minutes, obtaining a plot over that period for the wavelengths in question and determining the area under the curve, from which the rate of loss can be calculated. Clearly the smaller the area under the curve the smaller the loss. For UVA absorption wavelengths from 320 to 400, especially from 340 to 390 nm, are considered.

While any reduction in the loss of UV absorption is an advantage, it is generally desirable that the presence of the doped oxide should reduce the rate of UV absorption by an amount of at least a 5%, preferably at least 10%, more preferably at least 15%, especially at least 20% and most preferably at least 40%.

The compositions of the present invention for cosmetics use may be, for example, lipsticks, skin anti-ageing compositions in the form of, for example, creams, including anti-wrinkle formulations, exfoliating preparations including scrubs, creams and lotions, skin lightening compositions in the form of, for example, face powders and creams, preparations for the hands including creams and lotions, moisturising preparations, compositions for protecting the hair such as conditioners, shampoos and hair lacquers as well as hair masks and gels, skin cleansing compositions including wipes, lotions and gels, eye shadow and blushers, skin toners and serums as well as washing products such as shower gels, bath products including bubble baths, bath oils, but, preferably, sunscreens. In this connection we should point out that the expression "cosmetic UV sunscreen composition", as used herein, includes any composition applied to the skin which may leave a residue on the skin such as some washing products. Compositions of the present invention may be employed as any conventional formulation providing protection from UV light. The composition may also be a pharmaceutical composition suitable for topical application. Such compositions are useful, in particular, for patients suffering from disorders of the skin which are adversely affected by UV light such as those giving rise to polymorphous light eruptions.

Organic sunscreen agents which can be used in the compositions of the present invention include any conventional sunscreen agent which gives protection against UV light while if there is no other component which is degraded by TiO₂ and/or ZnO the sunscreen agent is itself degraded by TiO₂ and/or ZnO. Suitable sunscreen agents are listed in the IARC Handbook of Cancer Prevention, vol. 5, Sunscreens, published by the International Agency for Research on Cancer, Lyon, 2001 and include:
(a) Para-aminobenzoic acids (PABA), (UVB absorbers) esters and derivatives thereof, for example amyldimethyl-; ethyldihydroxypropyl-; ethylhexyl dimethyl-; ethyl-; glyceryl-; and 4-bis-(polycthoxy)- PABA.
(b) Cinnamates (UVB) especially esters including methyl cinnamate esters and methoxycinnamate esters such as octylmethoxy cinnamate, ethyl methoxycinnamate, especially 2-ethylhexyl paramethoxycinnamate, isoamyl p-methoxy cinnamate, or a mixture thereof with diisopropyl cinnamate, 2-ethoxyethyl -4-methoxycinnamate, DEA-methoxycinnamate (diethanolamine salt of para-methoxy hydroxycinnamate) or a,β-di-(para-methoxycinnamoyl)-α'-(2-ethylhexanoyl)-glycerin, as well as diisopropyl methylcinnamate;
(c) benzophenones (UVA) such as 2,4-dihydroxy-; 2-hydroxy-4-methoxy; 2,2'-dihydroxy-4,4'-dimethoxy-; 2,2'-dihydroxy-4-methoxy-;' 2,2',4,4'-tetrahydroxy-; and 2-hydroxy-4-methoxy-4'-methyl-benzophenones, benzenesulphonic acid and its sodium salt; sodium 2,2'-dihydroxy-4,4'-dimethoxy-5-sulphobenzophenone and oxybenzone;
(d) dibenzoylmethanes (UVA) such as butyl methoxydibenzoyl methane, especially 4-tert-butyl-4'methoxydibenzoylmethane;
(e) 2-phenylbenzimidazole-5 sulfonic acid UVB and phenyldibenzimidazole sulfonic acid and their salts;
(f) alkyl-β,β-diphenylacrylates (UVB) for example alkyl α-cyano-β, β-diphenylacrylates such as octocrylene;
(g) triazines (UVB) such as 2,4,6-trianilino-(p-carbo-2-ethyl-hexyl-1-oxy)-1,3,5 triazine as well as octyl triazone e.g. ethylhexyltriazone and diethylhexyl butamido triazone.
(h) camphor derivatives (generally UVB) such as 4-methylbenzylidene and 3-benzylidene- camphor and terephthalylidene dicamphor sulphonic acid (UVA), benzylidene camphor sulphonic acid, camphor benzalkonium methosulphate and polyacrylamidomethyl benzylidene camphor;
(i) organic pigment sunscreening agents such as methylene bis-benzotriazole tetramethyl butylphenol;
(j) silicone based sunscreening agents such as dimethicodiethyl benzal malonate.
(k) salicylates (UVB) such as dipropylene glycol-; ethylene glycol-, ethylhexyl-, isopropylbenzyl-, methyl-, phenyl-, 3,3,5-trimethyl- and TEA-salicylate (compound of 2-hydroxybenzoic acid and 2,2'2"-nitrilotris (ethanol));
(l) anthranilates (UVA) such as menthyl anthranilate
as well as bisymidazylate (UVA), dialkyl trioleate (UVB), 5-methyl-2-phenylbenzoxazole (UVB) and urocanic acid (UVB).

Some compounds are effective for both UVA and UVB. These include anisotriazine, methylene bisbenzotriazolyl tetramethylbutyl- phenol and drometrizole trisiloxane (Mexoryl XL).

The organic sunscreen agent(s) are typically present in the compositions at a concentration from 0.1 to 20%, preferably 1 to 10%, and especially 2 to 5%, by weight based on the weight of the composition.

In the compositions, which are generally aqueous, the metal oxides are preferably present at a concentration of about 0.5 to 20 % by weight, preferably about 1 to 10 % by weight and more preferably about 3 to 8 % by weight, in particular about 4 to 7%, such as 4 to 6% for example about 5%, by weight.

The compositions may be in the form of, for example, lotions, typically with a viscosity of 4000 to 10,000 mPas, e.g. thickened lotions, gels, vesicular dispersions, creams, typically a fluid cream with a viscosity of 10,000 to 20,000 mPas or a cream of viscosity 20,000 to 100,000 mPas, milks, powders, solid sticks, and may be optionally packaged as aerosols and provided in the form of foams or sprays.

The compositions may contain any of the ingredients used in such formulations including fatty substances, organic solvents, silicones, thickeners, liquid and solid emollients, demulcents, other UVA, UVB or broad-band sunscreen agents, antifoaming agents, antioxidants such as butyl hydroxy toluene, buffers such as lactic acid with a base such as triethanolamine or sodium hydroxide, plant extracts such as Aloe vera, cornflower, witch hazel, elderflower and cucumber, activity enhancers, moisturizing agents, and humectants such as glycerol, sorbitol, 2-pyrrolidone-5-carboxylate, dibutylphthalate, gelatin and polyethylene glycol, perfumes, preservatives, such as para-hydroxy benzoate esters, surface-active agents, fillers and thickeners, sequesterants, anionic, cationic, nonionic or amphoteric polymers or mixtures thereof, propellants, alkalizing or acidifying agents, colorants and powders, including metal oxide pigments with a particle size of from 100 nm to 20000 nm such as iron oxides along with conventional (undoped) TiO₂ and ZnO.

Other ingredients of cosmetic compositions, for example some surface-active agents may have the effect of degrading certain sunscreen agents in the presence of UV light. Also TiO₂ and ZnO are known to degrade certain organic sunscreens such as oxybenzone as well as antioxidants such as vitamins e.g. vitamins A, B, C and E, and also anti-ageing factors such as niacinamide, retinoids and coenzyme MEQ10 etc. It will be appreciated that it is particularly useful to use the doped TiO₂ and/or ZnO and/or reduced ZnO with such sunscreens. This is because TiO₂ and ZnO do generally have a positive UV absorptive effect. Thus by using the doped TiO₂ and/or ZnO and/or reduced ZnO it may be possible to use less antioxidant or make the formulation longer lasting.

The organic solvents are typically selected from lower alcohols and polyols such as ethanol, isopropanol, propylene glycol, glycerin and sorbitol as well as methylene chloride, acetone, ethylene glycol monoethyl ether, diethylene glycol monobutyl ether, diethylene glycol mono-ethyl, ether, dimethyl sulphoxide, dimethyl formamide and tetrahydrofuran.

The fatty substances may consist of an oil or wax or mixture thereof, fatty acids, fatty acid esters, fatty alcohols, vaseline, paraffin, lanolin, hydrogenated lanolin or acetylated lanolin, beeswax, ozokerite wax and paraffin wax.

The oils are typically selected from animal, vegetable, mineral or synthetic oils and especially hydrogenated palm oil, hydrogenated castor oil, vaseline oil, paraffin oil, Purcellin oil, silicone oil such as polydimethyl siloxanes and isoparaffin.

The waxes are typically animal, fossil, vegetable, mineral or synthetic waxes. Such waxes include beeswax, Carnauba, Candelilla, sugar cane or Japan waxes, ozokerites, Montan wax, microcrystalline waxes, paraffins or silicone waxes and resins.

The fatty acid esters are, for example, isopropyl myristate, isopropyl adipate, isopropyl palmitate, octyl palmitate, C₁₂-C₁₅ fatty alcohol benzoates ("FINSOLV TN" from FINETEX), oxypropylenated myristic alcohol containing 3 moles of propylene oxide ("WITCONOL APM" from WITCO), capric and caprylic acid triglycerides ("MIGLYOL 812" from HULS).

The compositions may also contain thickeners such as cross-linked or non cross-linked acrylic acid polymers, and particularly polyacrylic acids which are cross-linked using a polyfunctional agent, such as the products sold under the name "CARBOPOL" by the company GOODRICH, cellulose or derivatives thereof such as methylcellulose, hydroxymethylcellulose, hydroxypropyl methylcellulose, sodium salts of carboxymethyl cellulose, or mixtures of cetylstearyl alcohol and oxyethylenated cetylstearyl alcohol containing, say, 33 moles of ethylene oxide.

Desirably, the weight ratio of water-dispersible titanium dioxide to oil-dispersible titanium dioxide is from 1:4 to 4:1, preferably from 1:2 to 2:1 and ideally about equal weight proportions.

Suitable emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, caster oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate.

Suitable propellants include propane, butane, isobutane, dimethyl ether, carbon dioxide, nitrous oxide.

Suitable powders include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

When the compositions of the present invention are sunscreens they may be in the form of, for example, suspensions or dispersions in solvents or fatty substances or as emulsions such as creams or milks, in the form of ointments, gels, solid sticks or aerosol foams. The emulsions, which can be oil-in-water or water-in-oil emulsions, may further contain an emulsifier including anionic, nonionic, cationic or amphoteric surface-active agents; for a water-in-oil emulsion the HLB is typically from 1 to 6 while a larger value i.e greater than 6 is desirable for an oil-in-water emulsion. Generally, water amounts to up to 80%, typically 5 to 80%, by volume. Specific emulsifiers which can be used include sorbitan trioleate, sorbitan tristearate, glycerol monooleate, glycerol monostearate, glycerol monolaurate, sorbitan sesquioleate, sorbitan monooleate, sorbitan monostearate, polyoxyethylene (2) stearyl ether, polyoxyethylene sorbitol beeswax derivative, PEG 200 dilaurate, sorbitan monopalmitate, polyoxyethylen (3.5) nonyl phenol, PEG 200 monostearate, sorbitan monostearate, sorbitan monolaurate, PEG 400 dioleate, polyoxyethylene (5) monostearate, polyoxyethyene (4) sorbitan monostearate, polyoxyethylene (4) lauryl ether, polyoxyethylene (5) sorbitan monooleate, PEG 300 monooleate, polyoxyethylene (20) sorbitan tristearate, polyoxyethylene (20) sorbitan trioleate, polyoxyethylene (8) monostearate, PEG 400 monooleate, PEG 400 monostearate, polyoxyethylene (10) monooleate, polyoxyethylene (10) stearyl ether, polyoxyethylene (10) cetyl ether, polyoxyethylene (9.3) octyl phenol, polyoxyethylene (4) sorbitan monolaurate, PEG 600 monooleate, PEG 1000 dilaurate, polyoxyethylene sorbitol lanolin derivative, polyoxyethylene (12) lauryl ether, PEG 1500 dioleate, polyoxyethylene (14) laurate, polyoxyethylene (20) sorbitan monostearate, polyoxyethylene (20) sorbitan monooleate, polyoxyethylene (20) stearyl ether, polyoxyethylene (20) sorbitan monopalmitate, polyoxyethylene (20) cetyl ether, polyoxyethylene (25) oxypropylene monostearate, polyoxyethylene (20) sorbitol monolaurate, polyoxyethylene (23) lauryl ether, polyoxyethylene (50) monostearate, and PEG 4000 monostearate. Alternatively the emulsifier can be a silicone surfactant, especially a dimethyl polysiloxane with polyoxyethylene and/or polyoxypropylene side chains, typically with a molecular weight of 10,000 to 50,000, especially cyclo-methicone and dimethicone copolyol. They may also be provided in the form of vesicular dispersions of ionic or nonionic amphiphilic lipids prepared according to known processes.

It can be advantageous to use both a water-dispersible and an oil-dispersible titanium dioxide or zinc oxide, at least one of which is doped or, in the case of zinc oxide, reduced. It has been found that when an emulsion is spread on the skin it has a tendency to break down into oily and non-oily areas. When the water evaporates the oil-dispersible particles will tend to be in the oily areas thus leaving areas unprotected. This can be avoided by having both hydrophilic and hydrophobic particles in the emulsion so that some are retained in hydrophilic areas and others in hydrophobic areas.

Water-dispersible particles can be uncoated or coated with a material to impart a hydrophilic surface property to the particles. Examples of such materials include aluminium oxide and aluminum silicate. Oil-dispersible particles which exhibit a hydrophobic surface property, are suitably coated with metal soaps such as aluminium stearate, aluminium laurate or zinc stearate, or with organosilicone compounds.

Although the present invention has particular utility for UV sunscreen compositions which may contain TiO₂ and/or ZnO, it extends to all compositions which may contain TiO₂ and/or ZnO. Thus the present invention also has applicability for polymeric compositions. By "a polymeric composition" as used herein is meant a composition which comprises one or more polymeric materials. The composition can be solid or liquid.

The composition of the present invention will contain TiO₂ and/or ZnO which has not been doped or, in the case of ZnO, reduced. Typically such undoped TiO₂/ZnO will be present as pigment, generally having a particle size of at least 100 nm.

Typical solid materials include polymeric solids including three dimensional objects, films and fibres as well as textiles and fabrics e.g. clothing and netting made from woven and non-woven fibres as well as foamed articles. Three-dimensional objects include those made by melt-forming processes including extruded and moulded articles. Typical articles to which the present invention may be applied include generally external household and building materials including blinds and plastics curtains, trellises, pipes and guttering, cladding and facings such as soffit board and plastics roofing material which can be profiled as with corrugated sheeting, doors and windows frames. Other articles include advertising hoardings and the like e.g. advertising boards on vehicle sides as well as vehicle bodies and body parts including bumpers for cars, buses and trucks as well as roofs which can be used also for boats, as well as superstructures and hulls for boats and also bodies for lawnmowers and tractors and yachts, along with containers such as bottles, cans, drums, buckets and oil and water storage containers. Other objects include garden furniture.

Films to which the present invention can be applied include self supporting as well as non-self supporting films such as coatings. Self-supporting films to which the present invention applies include photographic films, packaging film and plastic film bearing indicia, typically as advertising film, which can also be applied over advertising hoardings. Such films can contain one or more customary ingredients for such products. Thus photographic film will contain one or more dyes or dye couplers and, optionally, a silver halide.

Coating compositions are typically paints and varnishes which contain a polymer either as the active ingredient as in some varnishes or as a support as in paints along with furniture polishes, waxes and creams; they can be aqueous or non aqueous i.e. contain an organic solvent. This coating composition can be in the form of a waterproofing agent. These coating compositions can contain one or more customary ingredients for such products.

The polymers which can be used in the compositions of the present invention include natural and synthetic polymers which may be thermoplastic or thermosetting.

The suitable polymers which may be homopolymers or copolymers which can be random, block or graft copolymers; the polymers can be crosslinked. Such polymers may be saturated or unsaturated. Typical polymers include alkylene polymers such as ethylene and propylene polymers, typically homopolymers, including polyethylene foams, including PTFE, siloxane and sulphide polymers, polyamides such as nylon, polyesters, acrylate and methacrylate polymers e.g. poly(methyl methacrylate) as well as PET, polyurethanes, including foams, vinyl polymers such as styrene polymers e.g. ABS, including polystyrene foam vinyl chloride polymers and polyvinyl alcohol. Fluorinated polymers such as PTFE and polyvinylidene fluoride can be used. The polymers can be thermosetting as with epoxy resins as well as phenolic, urea, melamine and polyester resins

Natural polymers which can be used include cellulosic polymers, as in paper including starch, polysaccharides, lignins, and polyisoprenes such as natural rubbers.

Typical polymers for different applications include the following: (a) polyester, polyamide e.g. nylon, acrylics for fibres and fabrics; (b) polyester, polyvinyl chloride, polyethylene, polypropylene for bottles and the like; (c) polyethylene, polypropylene, polyvinyl chloride for film (non active such as packaging).

The polymeric compositions can contain the usual additional ingredients characteristic for the composition in question including inorganic and organic pigments, including "ordinary" TiO₂ and/or ZnO, fillers and extenders as well as light stabilisers, typically hindered amine stabilisers. The additional ingredients may themselves be susceptible to attack, with the degraded components potentially causing degradation of the polymer or of other components of the composition.

Reference may be made to a "physical factor", by which is meant a measurable value of a physical property of the composition which is adversely affected by UV light. Examples of physical properties which may be adversely affected by TiO₂ and/or ZnO induced free radical attack include degradation and, in consequence, strength, colour change e.g. for paints and textiles and photographic stability e.g. for photographic films.

Thus if the rate of deterioration of a physical factor is X then the amount of the component(s) which is degraded possesses a said rate of deterioration ofY where Y is greater than X, preferably by at least 5%, and the amount of doped TiO₂ and/or ZnO and/or reduced ZnO reduces the said rate of loss from Y to X.

The rate of colour change can be determined by illuminating a sample of the composition with and without the doped TiO₂ or ZnO or reduced ZnO with sunlight or visible light and measuring the spectral response of the composition over a given period and determining the change in wavelength emitted. Accelerated ageing tests using, for example a Fadeometer, can be used for this purpose.

The rate of loss of strength of an article of the present invention can be determined in a similar manner by measuring tensile properties such as the elongation at break or Young's modulus using standard equipment such as an Instron tester; again an accelerated ageing procedure is beneficial.

While any reduction in the wavelength change or other physical factor is an advantage, it is generally desirable that the presence of the doped oxide reduce the rate of change by an amount of at least 5%, preferably at least 10%, more preferably at least 15%, especially at least 20% and most preferably at least 40%.

In the polymeric compositions the metal oxides are preferably present at a concentration of about 0.5 to 20 % by weight, preferably about 1 to 10 % by weight and more preferably about 3 to 8 % by weight.

The dopant for the oxide particles is preferably manganese, which is especially preferred, e.g. Me²⁺ but especially Mn³⁺,vanadium, for example V³⁺ or V⁵⁺, chromium and iron but other metals which can be used include nickel, copper, tin, aluminium, lead, silver, zirconium, zinc, cobalt, gallium, niobium, for example Nb⁵⁺, antimony, for example Sb³⁺, tantalum, for example Ta⁵⁺, strontium, calcium, magnesium, barium, molybdenum, for example Mo³⁺, Mo⁵⁺ or Mo⁶⁺ as well as silicon. Manganese is preferably present as Mn³⁺, cobalt as Co²⁺, tin as Sn⁴⁺ as well as Mn²⁺. These metals can be incorporated singly or in combination of 2 or 3 or more. Further details of these doped oxides can be found in WO99/60994 as well as WO01/40114.

The optimum amount of the second component in the host lattice may be determined by routine experimentation but it is preferably low enough so that the particles are not coloured. Amounts as low as 0.1 mole % or less, for example 0.05 mole %, or as high as 1 mole % or above, for example 5 mole % or 10 mole %, can generally be used. Typical concentrations are from 0.5 to 2 mole % by weight.

These particles can be obtained by any one of the standard processes for preparing doped oxides and salts. Thus they can be obtained by a baking technique by combining particles of a host lattice (TiO₂/ZnO) with a second component in the form of a salt such as a chloride or an oxygen-containing anion such as a perchlorate or a nitrate, in solution or suspension, typically in solution in water, and then baking it, typically at a temperature of at least 300°C. Other routes which may be used to prepare the doped materials include a precipitation process of the type described in J. Mat. Sci. (1997) 36, 6001-6008 where solutions of the dopant salt and of an alkoxide of the host metal (Ti/Zn) are mixed, and the mixed solution is then heated to convert the alkoxide to the oxide. Heating is continued until a precipitate of the doped material is obtained. Further details of preparation can be found in the aforesaid patent specifications.

The rutile form of titania is known to be more photostable than the anatase form and is therefore preferred.

Reduced zinc oxide particles (i.e. particles which possess an excess of zinc ions relative to the oxygen ions) may be readily obtained by heating zinc oxide particles in a reducing atmosphere to obtain reduced zinc oxide particles which absorb UV light, especially UV light having a wavelength below 390 nm, and re-emit in the green, preferably at about 500 nm. It will be understood that the reduced zinc oxide particles will contain reduced zinc oxide consistent with minimising migration to the surface of the particles of electrons and/or positively charged holes such that when said particles are exposed to UV light in an aqueous environment the production of hydroxyl radicals is substantially reduced as discussed above.

The reducing atmosphere can be air with a reduced oxygen content or an increased hydrogen content but is preferably a mixture of hydrogen and an inert gas such as nitrogen or argon. Typically the concentration of hydrogen is from 1 to 20%, especially 5 to 15%, by volume, with the balance inert gas, especially nitrogen. A preferred reducing atmosphere is about 10% hydrogen and about 90% nitrogen by volume. The zinc oxide is heated in this atmosphere at, say, 500° to 1000°C, generally 750 to 850°C, for example about 800°C, for 5 to 60 minutes, generally 10 to 30 minutes. Typically it is heated to about 800°C for about 20 minutes.

It is believed that the reduced zinc oxide particles possess an excess of Zn²⁺ ions within the absorbing core. These are localised states and as such may exist within the band gap. A further discussion of this can be found in WO 99/60994.

The average primary particle size of the particles is generally from about 1 to 200 nm, for example about 1 to 150 nm, preferably from about 1 to 100 nm, more preferably from about 1 to 50 nm and most preferably from about 20 to 50 nm. The particle size is preferably chosen to avoid colouration of the final product. Thus nanoparticles are frequently used. However, in one embodiment slightly larger particles for example from 100 to 500 nm, typically 100 to 400 or 450 mm especially from 150 to 300 nm and particularly 200 to 250 nm, can be employed. These provide good coverage of, for example, skin imperfections without unacceptable skin whitening.

Where particles are substantially spherical then particle size will be taken to represent the diameter. However, the invention also encompasses particles which are non-spherical and in such cases the particle size refers to the largest dimension.

The oxide particles used in the present invention may have an inorganic or organic coating. For example, the particles may be coated with oxides of elements such as aluminium, zirconium or silicon, especially silica. The particles of metal oxide may also be coated with one or more organic materials such as polyols, amines, alkanolamines, polymeric organic silicon compounds, for example, RSi[{OSi(Me)₂}xOR¹]₃ where R is C₁-C₁₀ alkyl, R¹ is methyl or ethyl and x is an integer of from 4 to 12, hydrophilic polymers such as polyacrylamide, polyacrylic acid, carboxymethyl cellulose and xanthan gum or surfactants such as, for example, TOPO. Such coatings can have the effect of masking, at least to some extent, any colour which the doped particles may have.

## Claims

1. A composition which comprises an ingredient which is adversely affected by UV light in the presence of TiO₂ and/or ZnO, TiO₂ and/or ZnO doped with another element and/or reduced ZnO, and wherein the composition contains TiO₂ and/or ZnO which is not doped or reduced.

2. A composition according to claim 1 wherein the said another element is manganese, preferably Mn³⁺, vanadium, chromium or iron.

3. A composition according to any one of the preceding claims wherein the said another element is present in an amount from 0.5 to 10 mole %.

4. A composition according to any one of the preceding claims which comprises doped titanium dioxide, preferably in rutile form.

5. A composition according to any one of the preceding claims wherein doped and/or undoped TiO₂ and/or ZnO therein is coated with inorganic or organic coating.

6. A composition according to claim 5 wherein the inorganic coating is an oxide of aluminium, zirconium or silicon, and the organic coating is one or more of an organic material selected from polyol, amine, alkanolamine, polymeric organic silicon compound, hydrophilic polymer and surfactant.

7. A composition according to any one of the preceding claims which comprises 0.5 to 20 % by weight of the doped TiO₂ or ZnO or reduced ZnO.

8. A composition according to any one of the preceding claims wherein the doped or reduced oxide has a particle size from 1 to 200 nm.

9. A composition according to any one of claims 1 to 7 wherein the doped or reduced oxide has a particle size from 100 to 500 nm.

10. A composition according to any one of the preceding claims wherein the TiO₂ and/or ZnO which is not doped has a particle size of at least 100 nm.

11. A composition according to any one of the preceding claims which is a UV sunscreen composition or other composition which is suitable for cosmetic use.

12. A composition according to claim 11 having a rate of loss of UV absorption at least 5% less than that of a composition having the same formulation except that it does not contain the said TiO₂ and/or ZnO doped with another element or the said reduced zinc oxide.

13. A composition according to any one of the preceding claims which contains a UV sunscreen agent which is adversely affected by TiO₂ and/or ZnO.

14. A composition according to any one of claims 11 to 13 wherein the organic sunscreen agent is a paraaminobenzoic acid, ester or derivative thereof, a methoxy cinnamate ester, a benzophenone, a dibenzylomethane, an alkyl-β,β-phenyl acrylate, a triazine, a camphor derivative, an organic pigment, a silicone based sunscreen agent or 2-phenylbenzimdazole-5 sulphonic acid, phenyldibenzimidazole sulphonic acid or salts thereof.

15. A composition according to any one of claims 12 to 14 wherein the rate of change of the ratio of the loss of UVA absorption to the loss of UVB absorption is less than that of a composition of the same formulation except that the TiO₂ and /or ZnO present is not doped.

16. A composition according to claim 15 wherein the rate of change of the ratio is greater because the rate of loss of UVA absorption is reduced.

17. A composition according to any one of claims 11 to 16 which comprises 0.1% to 20% by weight of organic sunscreen agent(s).

18. A composition according to any one of claims 11 to 17 which contains one or more of a fatty substance, organic solvent, silicone, thickener, demulcent, UVB sunscreen agent, antifoaming agent, moisturising agent, perfume preservative, surface activation filler, sequestrant, anionic, cationic, nonionic or amphoteric polymer, propellant, alkalising or acidifying agent, colorant, metal oxide pigment, vitamin, antioxidant, anti-ageing factor and stabilizer.

19. A composition according to any one of claims 11 to 18 which is in the form of a lotion, gel, dispersion, cream, milk, powder or solid stick.

20. A composition according to claim 18 or 19 which includes a water-dispersible and an oil-dispersible TiO₂ and/or ZnO.

21. A composition according to any one of claims 1 to 10 which comprises one or more polymeric materials.

22. A composition according to claim 21 wherein the ingredient which is adversely affected by TiO₂ and/or ZnO suffers a change in physical properties.

23. A composition according to claim 22 wherein the physical property is tensile strength or colour.

24. A composition according to any one of claims 21 to 23 wherein the polymeric material is thermoplastic or thermosetting.

25. A composition according to any one of claims 21 to 24 which is in the form of a three dimensional article or is in the form of a film, preferably a photographic film, or is in the form of a coating composition or which is in the form of a paint or varnish.

26. A composition according to any one of the preceding claims wherein the ingredient which is adversely affected by TiO₂ and/or ZnO is an ethylenically unsaturated compound or one possessing a labile hydrogen atom.

## Patentansprüche

1. Zusammensetzung, die einen Bestandteil enthält, der durch UV-Licht in Gegenwart von TiO₂ und/oder ZnO, von mit einem weiteren Element dotiertem TiO₂ und/oder ZnO und/oder von reduziertem ZnO nachteilig beeinflusst wird, wobei die Zusammensetzung TiO₂ und/oder ZnO enthält, das nicht dotiert oder reduziert ist.

2. Zusammensetzung nach Anspruch 1, wobei es bei dem weiteren Element um Mangan, vorzugsweise Mn³⁺, Vanadium, Chrom oder Eisen handelt.

3. Zusammensetzung nach einem der vorstehenden Ansprüche, wobei das weitere Element in einer Menge von 0,5 bis 10 Mol-% vorliegt.

4. Zusammensetzung nach einem der vorstehenden Ansprüche, die dotiertes Titandioxid, vorzugsweise in Rutilform, enthält.

5. Zusammensetzung nach einem der vorstehenden Ansprüche, wobei darin enthaltenes dotiertes und/oder undotiertes TiO₂ und/oder ZnO mit einem anorganischen oder organischen Überzug beschichtet sind.

6. Zusammensetzung nach Anspruch 5, wobei es sich beim anorganischen Überzug um ein Oxid von Aluminium, Zirkonium oder Silicium handelt und es sich beim organischen Überzug um ein oder mehrere organische Materialien handelt, die aus Polyolen, Aminen, Alkanolaminen, polymeren organischen Siliciumverbindungen, hydrophilen Polymeren und oberflächenaktiven Mitteln ausgewählt sind.

7. Zusammensetzung nach einem der vorstehenden Ansprüche, die 0,5 bis 20 Gew.-% dotiertes TiO₂ oder ZnO oder reduziertes ZnO umfasst.

8. Zusammensetzung nach einem der vorstehenden Ansprüche, wobei das dotierte oder reduzierte Oxid eine Teilchengröße von 1 bis 200 nm aufweist.

9. Zusammensetzung nach einem der Ansprüche 1 bis 7, wobei das dotierte oder reduzierte Oxid eine Teilchengröße von 100 bis 500 nm aufweist.

10. Zusammensetzung nach einem der vorstehenden Ansprüche, wobei das TiO₂ und/oder ZnO, das nicht dotiert ist, eine Teilchengröße von mindestens 100 nm aufweist.

11. Zusammensetzung nach einem der vorstehenden Ansprüche, bei der es sich um eine UV-Sonnenschutzzusammensetzung oder eine andere für kosmetische Zwecke geeignete Zusammensetzung handelt.

12. Zusammensetzung nach Anspruch 11 mit einer Rate des Verlustes der UV-Absorption, die um mindestens 5 % geringer ist als die einer Zusammensetzung mit der gleichen Formulierung, mit der Ausnahme, dass sie nicht das mit einem weiteren Element dotierte TiO₂ und/oder ZnO oder das reduzierte Zinkoxid enthält.

13. Zusammensetzung nach einem der vorstehenden Ansprüche, die ein UV-Sonnenschutzmittel enthält, das durch TiO₂ und/oder ZnO nachteilig beeinflusst wird.

14. Zusammensetzung nach einem der Ansprüche 11 bis 13, wobei es sich beim organischen Sonnenschutzmittel um p-Aminobenzoesäure, einen Ester oder ein Derivat davon, einen Methoxycinnamatester, ein Benzophenon, ein Dibenzylmethan, ein Alkyl-β,β-phenylacrylat, ein Triazin, ein Campherderivat, ein organisches Pigment, ein Sonnenschutzmittel auf Siliconbasis, 2-Phenylbenzimidazol-5-sulfonsäure oder Phenyldibenzimidazolsulfonsäure oder Salze davon handelt.

15. Zusammensetzung nach einem der Ansprüche 12 bis 14, wobei die Veränderungsrate des Verhältnisses des Verlustes der UVA-Absorption zum Verlust der UVB-Absorption geringer ist als die einer Zusammensetzung mit der gleichen Formulierung, mit der Ausnahme, dass das vorhandene TiO₂ und/oder ZnO nicht dotiert ist.

16. Zusammensetzung nach Anspruch 15, wobei die Veränderungsrate des Verhältnisses größer ist, da die Rate des Verlustes von UVA-Absorption verringert ist.

17. Zusammensetzung nach einem der Ansprüche 11 bis 16, die 0,1 bis 20 Gew.-% des oder der organischen Sonnenschutzmittel enthält.

18. Zusammensetzung nach einem Ansprüche 11 bis 17, die einen oder mehrere der Bestandteile Fettsubstanzen, organische Lösungsmittel, Silicone, Verdickungsmittel, Demulcentia, UVB-Sonnenschutzmittel, Antischaummittel, Feuchthaltemittel, Duftstoffe, Konservierungsmittel, Oberflächenaktivierungsfüllstoffe, Sequestriermittel, anionische, kationische, nicht-ionische oder amphotere Polymere, Treibmittel, Alkalisierungs- oder Ansäuerungsmittel, farbgebende Mittel, Metalloxidpigmente, Vitamine, Antioxidantien, Antiaging-Faktoren und Stabilisatoren enthält.

19. Zusammensetzung nach einem der Ansprüche 11 bis 18, die in Form einer Lotion, eines Gels, einer Dispersion, einer Creme, einer Milch, eines Pulvers oder eines festen Stiftes vorliegt.

20. Zusammensetzung nach Anspruch 18 oder 19, die ein in Wasser dispergierbares und ein in Öl dispergierbares TiO₂ und/oder ZnO umfasst.

21. Zusammensetzung nach einem der Ansprüche 1 bis 10, die ein oder mehrere polymere Materialien umfasst.

22. Zusammensetzung nach Anspruch 21, wobei der Bestandteil, der durch TiO₂ und/oder ZnO nachteilig beeinflusst wird, eine Veränderung seiner physikalischen Eigenschaften erfährt.

23. Zusammensetzung nach Anspruch 22, wobei es sich bei der physikalischen Eigenschaft um die Zugfestigkeit oder um die Farbe handelt.

24. Zusammensetzung nach einem der Ansprüche 21 bis 23, wobei das polymere Material thermoplastisch oder wärmehärtend ist.

25. Zusammensetzung nach einem der Ansprüche 21 bis 24, die in Form eines dreidimensionalen Gegenstands oder in Form eines Films, vorzugsweise eines photographischen Films, oder in Form einer Beschichtungszusammensetzung oder in Form eines Anstrichmittels oder Lackes vorliegt.

26. Zusammensetzung nach einem der vorstehenden Ansprüche, wobei es sich bei dem Bestandteil, der durch TiO₂ und/oder ZnO nachteilig beeinflusst wird, um eine ethylenisch ungesättigte Verbindung oder um eine Verbindung, die ein labiles Wasserstoffatom aufweist, handelt.

## Revendications

1. Composition comprenant un ingrédient qui est défavorablement affecté par la lumière UV en présence de TiO₂ et/ou de ZnO, TiO₂ et/ou de ZnO dopé avec un autre élément et/ou de ZnO réduit, et ladite composition contient du TiO₂ et/ou du ZnO qui n'est pas dopé ou réduit.

2. Composition selon la revendication 1, dans laquelle ledit autre élément est du manganèse, de préférence Mn³⁺, du vanadium, du chrome ou du fer.

3. Composition selon l'une quelconque des revendications précédentes, dans laquelle ledit autre élément est présent en une quantité de 0,5 à 10 % en moles.

4. Composition selon l'une quelconque des revendications précédentes, qui comprend du dioxyde de titane dopé, de préférence sous la forme de rutile.

5. Composition selon l'une quelconque des revendications précédentes, dans laquelle du ZnO et/ou du TiO₂ dopé et/ou non dopé est revêtu d'un revêtement inorganique ou organique.

6. Composition selon la revendication 5, dans laquelle le revêtement inorganique est un oxyde d'aluminium, de zirconium ou de silicium, et le revêtement organique est une ou plusieurs matières organiques choisies parmi un polyol, une amine, une alcanolamine, un composé organique polymère de silicium, un polymère hydrophile et un agent tensioactif.

7. Composition selon l'une quelconque des revendications précédentes, qui comprend de 0,5 à 20 % en poids du ZnO et/ou du TiO₂ dopé ou du ZnO réduit.

8. Composition selon l'une quelconque des revendications précédentes, dans laquelle l'oxyde dopé ou réduit a une.taille de particules de 1 à 200 nm.

9. Composition selon l'une quelconque des revendications 1 à 7, dans laquelle l'oxyde dopé ou réduit a une taille de particules de 100 à 500 nm.

10. Composition selon l'une quelconque des revendications précédentes, dans laquelle le TiO₂ et/ou le ZnO qui n'est pas dopé a une taille de particules d'au moins 100 nm.

11. Composition selon l'une quelconque des revendications précédentes, qui est une composition d'écran solaire filtrant les rayons UV ou une autre composition qui est appropriée à une utilisation cosmétique.

12. Composition selon la revendication 11, ayant un taux de perte d'absorption de rayons UV d'au moins 5 % inférieur à celui d'une composition ayant la même formulation, sauf qu'elle ne contient pas ledit TiO₂ et/ou ledit ZnO dopé avec un autre élément ou ledit oxyde de zinc réduit.

13. Composition selon l'une quelconque des revendications précédentes, qui contient un agent solaire filtrant les rayons UV qui est défavorablement affecté par du TiO₂ et/ou du ZnO.

14. Composition selon l'une quelconque des revendications 11 à 13, dans laquelle l'agent solaire organique est un acide para-aminobenzoïque, un ester ou un dérivé de celui-ci, un ester méthoxycinnamate, une benzophénone, un dibenzylométhane, un acrylate d'alkyl-β,β-phényle, une triazine, un dérivé du camphre, un pigment organique, un agent solaire à base de silicone ou de l'acide 2-phénylbenzimidazoyl-5-sulfonique ou de l'acide phényldibenzimidazoyle sulfonique, ou des sels de ceux-ci.

15. Composition selon l'une quelconque des revendications 12 à 14, dans laquelle le taux de changement du rapport de la perte d'absorption de rayons UVA à la perte d'absorption de rayons UVB est inférieur à celui d'une composition de la même formulation, sauf que le TiO₂ et/ou ZnO présent n'est pas dopé.

16. Composition selon la revendication 15, dans laquelle le taux de changement du rapport est plus élevé car le taux de perte d'absorption de rayons UVA est réduit.

17. Composition selon l'une quelconque des revendications 11 à 16, qui comprend de 0,1 % à 20 % en poids d'un ou plusieurs agents solaires organiques.

18. Composition selon l'une quelconque des revendications 11 à 17, qui contient un ou plusieurs éléments choisis parmi une substance grasse, un solvant organique, de la silicone, un épaississant, un adoucissant, un agent solaire filtrant les rayons UVB, un agent antimousse, un agent hydratant, un agent de conservation de parfum, une charge d'activation de surface, un séquestrant, un polymère anionique, cationique, non ionique ou amphotère, un propulseur, un agent alcalifiant ou acidifiant, un colorant, un pigment d'oxyde métallique, une vitamine, un anti-oxydant, un facteur antivieillissement et un stabilisant.

19. Composition selon l'une quelconque des revendications 11 à 18, qui est sous la forme d'une lotion, d'un gel, d'une dispersion, d'une crème, d'un lait, d'une poudre ou d'un bâtonnet solide.

20. Composition selon la revendication 18 ou 19, qui inclut du ZnO et/ou du TiO₂ dispersables dans l'eau et dispersables dans l'huile.

21. Composition selon l'une quelconque des revendications 1 à 10, qui comprend une ou plusieurs matières polymères.

22. Composition selon la revendication 21, dans laquelle l'ingrédient qui est défavorablement affecté par du TiO₂ et/ou du ZnO subit un changement de propriétés physiques.

23. Composition selon la revendication 22, dans laquelle la propriété physique est la résistance à la traction ou la couleur.

24. Composition selon l'une quelconque des revendications 21 à 23, dans laquelle la matière polymère est thermoplastique ou thermodurcissable.

25. Composition selon l'une quelconque des revendications 21 à 24, qui est sous la forme d'un article tridimensionnel ou qui est sous la forme d'un film, de préférence un film photographique, ou qui est sous la forme d'une composition de revêtement ou qui est sous la forme d'une peinture ou d'un vernis.

26. Composition selon l'une quelconque des revendications précédentes, dans laquelle l'ingrédient qui est défavorablement affecté par du TiO₂ et/ou du ZnO est un composé éthyléniquement insaturé ou un composé possédant un atome d'hydrogène labile.
